# EUROPEAN PATENT APPLICATION

(11) **EP 4 763 856 A1**
(43) Date of publication of application: **24.06.2026**
(21) Application number: 25158406.6
(22) Date of filing: 17.02.2025
(51) Int. Cl.: C07K 5/062

(54) **PROCESS FOR THE PREPARATION OF FMOC-GLY-THR [PSI (ME, ME) PRO]-OH**

(30) Priority: 17.12.2024 IN 202441099828
(71) Applicant: Divi's Laboratories Limited, 500 032 Hyderabad, Telangana (IN)
(72) Inventor: DIVI, Satchandra Kiran, 500032 Hyderabad (IN); RAO, Mysore Aswatha Narayana, 500032 Hyderabad (IN); PENDYALA, Srinivas Rao, 500032 Hyderabad (IN); DONEPUDI, Sridhar, 500032 Hyderabad (IN); BANDARUPALLI, Leela Maheswara Rao, 500032 Hyderabad (IN)
(74) Representative: v. Bezold & Partner Patentanwälte - PartG mbB

(57) **Abstract**

The present invention describes a process for the preparation of Fmoc-Gly-Thr [Psi (Me, Me) pro]-OH by reacting Fmoc-glycyl chloride with N, O-Bis(trimethylsilyl)-threonine trimethylsilyl ester, in the presence of a base, to obtain Fmoc-Gly-(O-trimethylsilyl)-threonine trimethylsilyl ester, which on acid hydrolysis gives Fmoc-Gly-Thr-OH. Cyclization of Fmoc-Gly-Thr-OH using 2-methoxy propene or 2,2-dimethoxypropane in the presence of pyridinium p-toluene sulfonate as the acid catalyst, in 2-methyltetrahydrofuran, results in Fmoc-Gly-Thr [Psi (Me, Me) pro]-OH. Direct cyclization of Fmoc-Gly-(O-trimethylsilyl)-L-Threonine -trimethylsilyl ester in a similar manner also gives Fmoc-Gly-Thr [Psi (Me, Me) pro]-OH.

## Description

### Field of Invention:

The present invention relates to an improved process for the preparation of Fmoc-Gly-Thr [Psi (Me, Me) pro]-OH, useful in peptide synthesis.

### Background of the Invention:

Fmoc-Gly-Thr [Psi (Me, Me) pro]-OH is the pseudoproline of Fmoc-Glycyl-threonine having the structure (I):

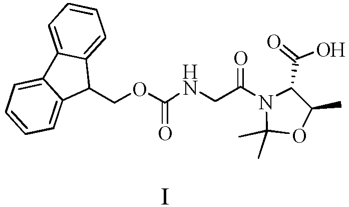

Oxazolidine and thiazolidine derivatives of protected dipeptides of serine, threonine and cysteine have structural similarity to proline. Hence, these derivatives are named "pseudoprolines." Because of their ability to disrupt secondary structure they prevent interchain aggregation during peptide synthesis. They enhance coupling efficiency and play an important role in the synthesis of peptides.

The U.S. Patent No. 7,612,209 B2 describes a process for the synthesis of pseudoproline dipeptides where N-protected amino acid is coupled with serine or threonine to obtain a dipeptide which is converted into ammonium salt in crystalline form. The ammonium salt of the dipeptide is reacted with an acid to release the dipeptide. In the next step, the ring closure of the free acid of the dipeptide is carried out in the presence of pyridinium p-toluene sulfonate (PPTS) as the acid catalyst. Although several reagents for the ring closure are mentioned, only a process using 2,2-dimethoxypropane (DMP) for the ring closure is described. In the above process, the amino acid is activated prior to its reaction with serine or threonine by activating reagent selected from the group consisting of N, N-diispropylcarbodiimide (DIC)/N-hydroxysuccinimide (HOSu), DIC/pentaflurophenol, DIC/1-hydroxybenzotriazole (HOBT), N,N-dicyclohexylcarbodiimide (DCC)/HOSu, DCC/pentaflurophenol, DCC/HOBT, (3-dimethylamino propyl)-ethyl carbodiimide HCl (EDC.HCl)/HOSu, and O-benzotriazole N.N.N.N-tetramethyl uranium hexaflurophosphate (HBTU)/HOBT.

The U.S. Patent No. 8,153,815 B2 describes a similar process where the dipeptide is prepared by reacting the N-protected amino acid with serine or threonine using a water soluble carbodiimide as the activating agent and without isolating or crystalizing the dipeptide. The dipeptide was cyclized using 2,2-dimethylpropane in the presence of PPTS to give the required pseudoproline. The CN102942625 describes a process for preparing Fmoc-Gly-Thr [Psi (Me, Me) pro]-OH. (Scheme 1). Fmoc-Glycine is condensed with L-threonine benzyl ester using HBTU to obtain Fmoc-Gly-Thr-OBz which was reacted with 2,2-dimethoxypropane and PPTS followed by debenzylation using hydrogen and Pd/C to obtain Fmoc-Gly-Thr [Psi (Me, Me) pro]-OH.

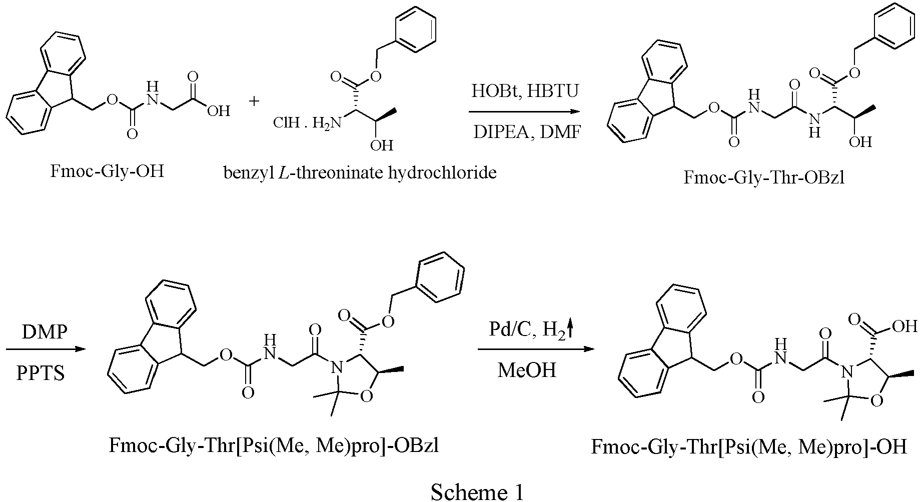

The CN103524595 reported a process (Scheme 2) in which N-hydroxysuccinimide ester of Fmoc-Glycine was condensed with L-threonine to obtain Fmoc-Gly-Thr-OH. It was reacted with 2,2-dimethoxypropane and methanesulfonic acid to obtain Fmoc-Gly-Thr [Psi (Me, Me) pro]-OH.

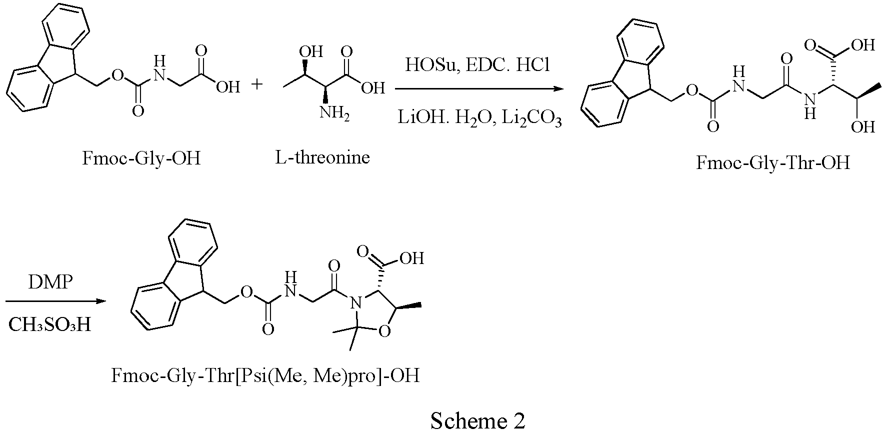

Although several processes are reported, there is a need for a new improved process for the preparation of Fmoc-Gly-Thr [Psi (Me, Me) pro]-OH.

### Summary of the Invention:

The present invention provides a novel process for the preparation of Fmoc-Gly-Thr [Psi- (Me, Me) Pro]-OH of formula I as given in the Scheme 3.

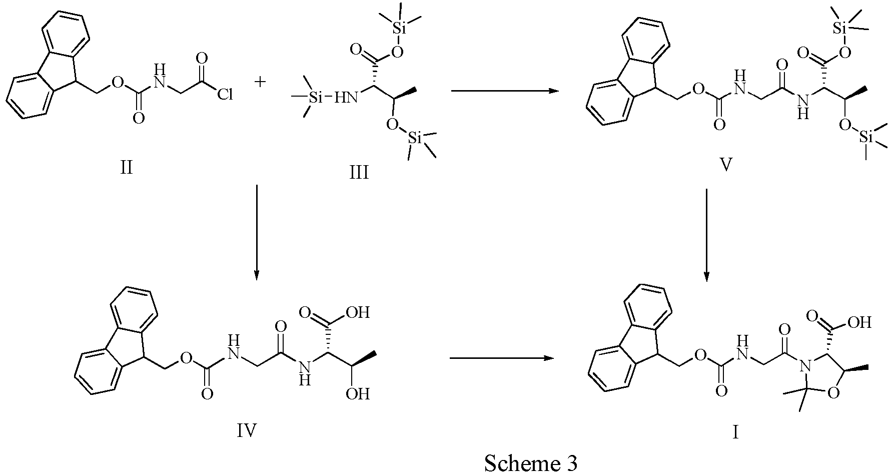

The process comprises,
(i) reacting Fmoc-glycyl chloride (II) with N, O-Bis(trimethylsilyl)-L-threonine trimethylsilyl ester (III), followed by acid hydrolysis, to give Fmoc-Gly-L-Thr-OH (IV),
(ii) cyclizing the Fmoc-Gly-Thr-OH (IV) using 2-methoxypropene or 2,2-dimethoxypropane, in the presence of an acid catalyst, to give Fmoc-Gly-Thr [Psi (Me, Me) pro]-OH (I).
(iii) optionally, reacting Fmoc-glycyl chloride (II) with N, O-Bis(trimethylsilyl)-L-threonine trimethylsilyl ester (III), to give Fmoc- Gly-(O-trimethylsilyl)-Threonine -trimethylsilyl ester (V), and
(iv) cyclizing the Fmoc-Gly-(O-trimethylsilyl)-Threonine-trimethylsilyl ester of the formula (V), using 2-methoxy propene or 2,2-dimethoxypropane, in the presence of an acid catalyst to give Fmoc-Gly-Thr [Psi (Me, Me) pro]-OH (I)

The process of the present invention gives Fmoc-Gly-Thr [Psi (Me, Me) pro]-OH (I) with high chemical and chiral purity without any racemization.

### Detailed Description of the Invention:

The present invention provides a novel process for the preparation of Fmoc-Gly-Thr [Psi (Me, Me) Pro]-OH of formula I (Scheme 3) which comprises the steps of:
(a) reacting Fmoc-glycyl chloride (II) with N, O-Bis(trimethylsilyl)-L-threonine trimethylsilyl ester (III) in the presence of a base, followed by acid hydrolysis, to give Fmoc-Gly-Thr-OH (IV),
(b) cyclizing Fmoc-Gly-Thr-OH (IV) into Fmoc-Gly-Thr [Psi (Me, Me) pro]-OH (I),
(c) optionally, reacting Fmoc-glycyl chloride (II) with N, O-Bis(trimethylsilyl)-L-threonine trimethylsilyl ester (III) in the presence of a base to give Fmoc-Gly-(O-trimethylsilyl)-L-threonine trimethylsilyl ester (V), and
(d) cyclizing the Fmoc-Gly-(O-trimethylsilyl)-L-threonine trimethylsilyl ester (V) to obtain Fmoc-Gly-Thr [Psi (Me, Me) pro]-OH (I)

The required starting compounds, Fmoc-glycyl chloride (II) (CAS No.: 103321-49-9) is prepared as reported in the Journal of Organic chemistry,1990, volume 55, pages 721-728 and the N, O-Bis(trimethylsilyl)-L-threonine trimethylsilyl ester (III) (CAS No.: 7537-02-2) is prepared as reported in the Journal of Organic chemistry, 1982, volume 47, pages 1324-1326.

Fmoc-amino acid chlorides are highly reactive and efficient coupling agents for the synthesis of peptides. The coupling process is very rapid, and under appropriate conditions, takes place without loss of chirality. Despite these advantages, Fmoc-amino acid chlorides are not widely used in the peptide synthesis. This is mainly due to difficulty in preparing stable Fmoc-amino acid chlorides of those amino acids which are having polar side chains, or those bearing tert-butyl protected side chains. Also, the Fmoc acid chlorides of tyrosine, serine, & threonine have poor stability.

However, Fmoc glycine is free from these drawbacks. It has no side chain and is non-chiral. It reacts with thionyl chloride and gives stable crystalline material that can be stored for a long time. Also, it can be prepared in very inexpensive manner. Hence, the present process selected Fmoc-glycyl chloride for coupling with threonine.

L-threonine is poorly soluble in organic solvents. Hence in most *prior art* it is dissolved in the aqueous alkaline solution of either sodium carbonate or a mixture of lithium hydroxide and lithium carbonate and reacted with the active ester to prepare a dipeptide where the active ester is dissolved in a water miscible solvent such as acetone, dimethylformamide or tetrahydrofuran. Dissolving the amino acid in an alkaline solution increases their susceptibility for racemization.

In the present process, L-threonine is used as its tri-silyl derivative, N, O-Bis(trimethylsilyl)-L-threonine trimethylsilyl ester (III). The tri-silyl threonine (III) is highly soluble in organic solvents such as toluene, methylene chloride, ethyl acetate, etc. Interestingly, the bulky trimethylsilyl group at the amine nitrogen enhances the nucleophilicity resulting in increased reactivity of the amine *(*Int. J. Peptide Protein Res. 33, 1989, 353-359). Furthermore, by blocking the chiral hydroxyl group and the carboxylic group as silyl derivative, possible side reactions are minimized.

The dipeptide, Fmoc-Gly-Thr-OH (IV) is prepared by reacting Fmoc-Gly-Cl (II) and N, O-Bis(trimethylsilyl)-L-threonine trimethylsilyl ester (III) in methylene chloride in the presence of a base such as diisopropylethylamine or triethylamine to neutralize the HCl liberated during the reaction. The reaction is carried out at 25°C to 30°C and requires about 2 to3 hours for the completion as monitored by TLC. After the reaction, the reaction mixture is first treated with water to remove the salts and then treated with 10% HCl for hydrolysing the silyl moieties. After removing the acidic phase, again water is added to the organic layer. The biphasic mixture is stirred for 4 to 8 hours which results in the precipitation of the product as a white solid. After filtering, the solid is washed with methylene chloride and dried at 50°C to 60°C for 12 to 15 hours resulting in Fmoc-Gly-Thr-OH (IV) in 80% yield with 98 % purity by HPLC.

The above reaction may also be conducted in 2-methyltetrahydrofuran as the solvent. However, unlike in 2-methyltetrahydrofuran, the reaction takes longer time, about 16 hours for the completion. When the reaction is conducted in toluene as the solvent, complete reaction is not observed and after treating with 10% HCl for hydrolysing the silyl moieties the product is obtained as a gummy material at the biphasic phase.

To prepare Fmoc-Gly-Thr [Psi (Me, Me) pro]-OH (I), the dipeptide, Fmoc-Gly-Thr-OH (IV) is suspended in 2-methyltetrahydrofuran which is a green solvent made from renewable lignocellulosic materials. To the suspension is added 0.15 equivalents of pyridinium p-toluene sulfonate (PPTS) and 4.0 equivalents of 2-methoxypropene.

At 25°C to 30°C, after about one hour, the reaction mixture becomes a clear solution, the reaction continued for 4 more hours. After workup, product is obtained in 86.3% yield with 93% HPLC purity containing 2.3% starting material. When the reaction is conducted at 40°C to 45°C, the reaction mixture becomes clear in about 15 min and after 2 hours, the reaction is almost complete. At 65°C to 70°C, an impurity of 0.2 to 0.5% is observed which remains at 0.12 to 0.18% even after the workup. At 50°C to 55°C reaction is complete in 2 hours and no impurity is observed. When the equivalents of 2-methoxypropene is reduced from 4.0 to 3.0 same results are obtained. After cooling the reaction mixture to room temperature, it is washed with water and brine. Another advantage of 2-methyltetrahydrofuran is being water immiscible, provides a clear phase separation from aqueous layer without forming emulsions or rag layers. After drying, solvent is removed under reduced pressure and the residue is crystallized using toluene and diisopropylethylamine mixture (1.5 vol: 2.0 vol) resulting in Fmoc-Gly-Thr [Psi (Me, Me) pro]-OH (I) as a white solid in 76.3% yield with HPLC purity of: 99.55%, enantiomeric excess (ee): 99.99%. The other isomer, Fmoc-Gly-D-Thr [Psi (Me, Me) pro]-OH was found to be 0.01% and no L-allo or D-allo-Threonine isomers are detected.

In addition to pyridinium p-toluene sulfonate (PPTS), other acid catalysts may also be used. When p-toluene sulfonic acid monohydrate is used as the catalyst, reaction time increased to about 15 hours. Methane sulfonic acid and boron trifluoride diethyl etherate catalysed reactions also takes longer time for the completion of the reaction. Thus, optimum results are obtained with PPTS.

The above reaction is conducted with 2,2-dimethoxypropane instead of 2-methoxypropene. Here, the reaction is carried out in 2-MeTHF using 0.20 equivalent PPTS and 8 equivalents of 2-dimethoxypropane at 65°C to 70°C for 4 to 5 hours. This resulted in Fmoc-Gly-Thr [Psi (Me, Me) pro]-OH (I) in 65.5% yields with 94.96%, purity by HPLC.

The reaction between Fmoc-Gly-Cl (II) and N, O-Bis(trimethylsilyl)-L-threonine trimethylsilyl ester (III) in the presence of diisopropylethyl amine results in Fmoc-Gly-(O-trimethylsilyl)-L-threonine-trimethylsilyl ester (V). The structure of (V) is confirmed by NMR. The reaction mixture also contains diisopropylethyl amine HCl salt. Because of the sensitivity of silyl compounds, the Fmoc-Gly-(O-trimethylsilyl)-threonine-trimethylsilyl ester(V) containing diisopropylethylamine Hydrochloride salt is directly reacted with 2-methoxypropene and PPTS in 2-MeTHF. Optimisation studies show that 1.0 equivalent PPTS and 10 equivalents of 2-methoxypropene are required for the best results. Although PPTS role is only catalytic in the reaction, the requirement of 1.0 equivalent suggests that it may be playing a role in de-silylation also and HCl salt being acidic may be playing a synergistic role. Under these conditions and at 65°C to 70°C reaction is complete in 2 hours. After workup the residue obtained is crystallized in toluene and di isopropyl ether mixture resulting in Fmoc-Gly-Thr [Psi (Me, Me) pro]-OH (I) in 70.5% yield with 98.51% purity by HPLC and 99.99% ee. The above reaction is also conducted using 2,2-dimethoxypropane replacing 2-methoxypropene. This resulted in 51.6% yield of Fmoc-Gly-Thr [Psi (Me, Me) pro]-OH (I) having 96.86% purity by HPLC.

When the reaction between Fmoc-Gly-Cl (II) and N, O-Bis(trimethylsilyl)-L-threonine trimethylsilyl ester (III) is conducted in the presence of triethylamine, it results in Fmoc-Gly-(O-trimethylsilyl)-L-threonine trimethylsilyl ester (V) and triethylamine HCl salt. When (V) containing triethylamine HCl salt is reacted with 2-methoxypropene, (I) is obtained in in 68.2% yield with 98.7% purity by HPLC.

The embodiments of the present invention are further described in the following examples, which are not intended in any way to limit the scope of the invention.

### Examples:

### Analytical Methods:

Chemical purity was determined using HPLC under the following conditions:
Column: XBridge C18, 150 x 4.6 mm, 5µm
Mobile phase: 0.1% TFA in water: acetonitrile (1:1), flow rate: 1.0 mL/min
Column Temperature: 10°C ± 2°C
Detection: 215 nm

Enantiomeric purity was determined using HPLC under the following conditions:
Column: Phenomenex lux cellulose-2, 250 x 4.6 mm, 5µm
Mobile phase:0.1% formic acid in water: acetonitrile (1:1), flow rate: 0.4 mL/min
Column Temperature: 30° C.
Detection: 215 nm

### Example-1: Preparation of Fmoc-Gly-Thr-OH (IV):

Fmoc-Gly-Cl (II) (21.4g, 67.2 mmol) was dissolved in methylene chloride (60 mL) and added to the reaction mixture containing N, O-Bis(trimethylsilyl)-L-threonine trimethylsilyl ester (III) (23.67g, 70.5 mmol, 1.05 eq.) and diisopropylethylamine (9.50g, 73.5 mmol, 1.10 eq.) in methylene chloride (60 mL) at 15°C to 20°C. The reaction mixture was warmed to 25°C to 30°C and stirred until the reaction is completed as monitored by TLC (2 to 3 hours). The reaction mixture was washed with water (200 mL) and then added10% aq. HCl (100 mL, con. HCl (10 mL) in 90 mL water) and stirred for 15 minutes. After removing the acidic phase, again water (200 mL) was added to the organic layer and the biphasic mixture was stirred for 4 to 8 hours resulting in the precipitation of the solid material. The solid material was filtered, washed with methylene chloride (60 mL) and dried at 50°C to 60°C for 12 to 15 hours to get Fmoc-Gly-Thr-OH (IV) as white solid. Yield: 21.6g (80%), purity by HPLC: 98.16%. FT-IR (ATR, cm⁻¹): 3356, 3305, 2892, 1702, 1667, 1640, 1572, 1538, 1484, 1466, 1451, 1445, 1411, 1375, 1341, 1297, 1272, 1252, 1228, 1193, 1156, 1109, 1076, 1052, 999, 970, 929, 861, 778, 757, 735; ¹HNMR (300 MHz, DMSO-*d₆*): δ 1.05 (d, 3H), 3.66-3.81 (m, 2H), 4.13-4.15 (m, 1H), 4.21-4.30 (m, 4H), 4.98 (broad s, 1H), 7.33 (t, 2H), 7.42 (t, 2H), 7.61 (t, 1H), 7.74 (t, 3H), 7.89 (d, 2H), 12.59 (broad s, 1H); ¹³CNMR (75 MHz, DMSO-*d₆*): δ 20.9, 43.8, 47.1, 58.0, 66.2, 66.8, 120.6, 125.7, 127.6, 128.1, 141.2, 144.3, 157.0, 170.0, 172.5; MASS (ESI +^{ve}): m/z 399.11 [M+H]⁺.

### Example-2: Preparation of Fmoc-Gly-Thr [Psi (Me, Me) pro]-OH (I):

Fmoc-Gly-Thr-OH (50g, 125.5 mmol) was suspended in 2-methyltetrahydrofuran (2-MeTHF) (500 mL), followed by addition of pyridinium p-toluene sulfonate (PPTS) (4.70g, 18.7 mmol, 0.15 eq.) and 2-methoxypropene (27.1g, 376 mmol, 3.0 eq.), stirred at 25°C to 30°C. for 10 minutes. The reaction mixture was heated to 50°C to 55°C and stirred for 1 to 2 hours. After completion of the reaction (monitored by TLC), the reaction mixture was cooled to 25°C to 30°C, washed with water (125 mL), brine solution (125 mL), dried using anhydrous sodium sulphate and concentrated under reduced pressure at 50 ±10°C. Crystallization in toluene and N, N-Diisopropylethylamine (DIPE) mixture (75 mL:100 mL) at ambient temp for 10 to 12 hours resulted in Fmoc-Gly-Thr[Psi (Me, Me)pro]-OH (I) as white solid. Yield: 42g (76.3%), purity by HPLC: 99.55%, enantiomeric excess (ee): 99.99%, The other isomer, Fmoc-Gly-D-Thr [Psi (Me, Me) pro]-OH was found to be 0.01% and no L-allo or D-allo-Threonine isomers are detected. FT-IR (ATR, cm⁻¹): 3417, 2938, 1740, 1715, 1636, 1517, 1441, 1410, 1384, 1372, 1281, 1256, 1229, 1191, 1161, 1126, 1108, 1080, 1049, 1012, 999, 988, 942, 907, 892, 858, 800, 778, 760, 742; ¹HNMR (300 MHz, CDCl₃): δ 1.43-1.49 (m, 3H), 1.58-1.75 (broad s, 6H), 3.78-4.00 (m, 2H), 4.10 (d, 1H), 4.20 (t, 1H), 4.25-4.45 (m, 3H), 5.00 (broad s, 1H), 6.02-6.07 (m, 1H), 7.30 (t, 2H), 7.39 (t, 2H), 7.57 (d, 2H), 7.75 (d, 2H); ¹³CNMR (75 MHz, CDCl₃): δ 18.8, 20.2, 23.9, 25.2, 27.0, 28.2, 42.9, 44.0, 46.9, 47.1, 64.7, 66.9, 67.5, 67.8, 73.0, 75.5, 94.8, 97.8, 120.0, 125.2, 127.1, 127.8, 141.3, 143.7, 143.7, 143.8, 156.7, 157.0, 166.2, 166.7, 171.8, 171.9; MASS (ESI +^{ve}): m/z 439.63 [M+H]⁺.

### Example-03: Preparation of Fmoc-Gly-Thr [Psi (Me, Me) pro]-OH (I):

Fmoc-Gly-Thr-OH (5.0g, 12.5 mmol, 1.0 eq.) was suspended in 2-MeTHF (50 mL), followed by addition of PPTS (0.63g, 2.50 mmol, 0.20 eq.) and 2,2-dimethoxypropane (10.46g, 100.4 mmol, 8.0 eq.), stirred at 25°C to 30°C. for 10 minutes. The reaction mixture was heated to 65°C to 70°C and stirred for 4 to 5 hours. Reaction mixture was cooled to 25°C to 30°C, followed by workup as described in Example-2, to obtain Fmoc-Gly-Thr [Psi (Me, Me) pro]-OH. Yield: 3.6g (65.5%), purity by HPLC: 94.96%,

### Example-04: Preparation of Fmoc-Gly-(O-trimethylsilyl)-L-threonine trimethylsilyl ester (V):

Fmoc-Gly-Cl (10.6g, 33.6 mmol) was dissolved in methylene chloride (30 mL) and added to the reaction mixture containing N, O-Bis(trimethylsilyl)-L-threonine trimethylsilyl ester (11.27g, 33.6 mmol, 1.0eq.) and diisopropylethylamine (4.76g, 36.8 mmol, 1.1 eq.) in methylene chloride (30 mL) at 15°C to 20°C. The reaction mixture was warmed to 25°C to 30°C and stirred until the reaction is complete as monitored by TLC (about 10 to 12 hours).

The reaction mixture was concentrated under reduced pressure to obtained 25.0g of semi solid residue containing Fmoc-Gly-(O-trimethylsilyl)-L-threonine trimethylsilyl ester. It also contained diisopropylethylamine hydrochloride salt.

¹HNMR (300 MHz, CDCl₃): δ 0.07 (m, 18H, -COOSiMe₃ and -OSiMe₃), 1.21 (broad s, 3H, -CH₃), 4.00 (broad, 2H, -CH₂-N), 4.20-4.34 (m, 3H, -CH-N & -CH₂-O), 4.40-4.42 (broad, 1H, -CH in Fmoc group), 4.57 (d, 1H, -CH-O), 7.25-7.38 (2x t, 6H), 7.53 (d, 2H), 7.72 (d, 2H) signals are related to Fmoc-Gly-(O-trimethylsilyl)-L-threonine-trimethylsilyl ester and 1.44 (d, 6H), 1.51-1.57 (m, 9H), 3.05-3.11 (m, 2H), 3.59-3.70 (m, 2H), 11.1 (s, 1H) signals are related to DIPEA. HCl

The purity of Fmoc-Gly-(O-trimethylsilyl)-L-threonine-trimethylsilyl ester could not be measured by HPLC as it underwent desilylation in the mobile phase to give the dipeptide, Fmoc-Gly-L-Thr-OH whose purity was found to be 98.84%.

Because of the sensitivity of silyl compounds, the Fmoc-Gly-(O-trimethylsilyl)-L-threonine-trimethylsilyl ester obtained as above which also contains diisopropylethylamine hydrochloride salt was taken to the next stage. Besides, the diisopropylethylamine hydrochloride being weakly acidic is compatible with the next stage reaction which uses acidic catalyst for preparing pseudoproline.

### Example-05: Preparation of Fmoc-Gly-Thr [Psi (Me, Me) pro]-OH (I):

Fmoc-Gly-(O-trimethylsilyl)-L-threonine-trimethylsilyl ester (25g) obtained from Example-4 (from Fmoc-Gly-Cl,10.6g, 33.6 mmol) was suspended in 2-MeTHF (180 mL) to which was added PPTS (8.44g, 33.6 mmol, 1.0 eq.) and 2-methoxy propene (24.23g, 10.0 eq.), stirred at 25°C to 30°C for 10 minutes. The reaction mixture was heated to 65°C to 70°C and stirring continued for 1 to 2 hours. After completion of the reaction (monitored by TLC), the reaction mixture was cooled to 25°C to 30°C, washed with water (50 mL), brine solution (50 mL), dried using anhydrous sodium sulphate and concentrated under reduced pressure at 50 ±10°C. After crystallization from toluene and di isopropyl ether mixture (75 mL:100 mL), Fmoc-Gly-Thr [Psi (Me, Me) pro]-OH (I) was obtained in 70.5% yield having 98.51% purity by HPLC and 99.99% ee by HPLC. FT-IR (ATR, cm⁻¹): 3418, 2989, 1741, 1715, 1636, 1519, 1442, 1411, 1384, 1372, 1281, 1256, 1231, 1192, 1163, 1126, 1109, 1080, 1050, 1013, 999, 989, 942, 908, 892, 859, 800, 778, 760, 742; ¹HNMR (300 MHz, CDCl₃): δ 1.44-1.49 (m, 3H), 1.59-1.75 (broad s, 6H), 3.79-3.86 (dd, 1H), 3.92-3.99 (dd, 1H), 4.10 (d, 1H), 4.19 (t, 1H), 4.34-4.42 (m, 3H), 6.07-6.09 (m, 2H), 7.29 (t, 2H), 7.39 (t, 2H), 7.57 (d, 2H), 7.75 (d, 2H); ¹³CNMR (75 MHz, CDCl₃): δ 18.8, 20.1, 23.9, 25.2, 26.9, 28.2, 42.9, 44.0, 46.9, 47.1, 64.7, 66.9, 67.4, 67.8, 73.0, 75.5, 94.8, 97.8, 120.0, 125.2, 127.1, 127.8, 141.3, 143.6, 143.7, 143.8, 156.7, 157.0, 166.0, 166.8, 171.8, 171.9; MASS (ESI +^{ve}): m/z 439.10 [M+H]⁺ and 461.10 [M+Na]⁺.

### Example-06: Preparation of Fmoc-Gly-Thr [Psi (Me, Me) pro]-OH (I):

The above example-05 was repeated by replacing 2-methoxypropene with 2,2-dimethoxy propane. After crystallization from methanol/toluene/di isopropyl ether mixture, Fmoc-Gly-Thr [Psi (Me, Me) pro]-OH was obtained in 51.6% yield having 96.86% purity by HPLC.

### Example-07: Preparation of Fmoc-Gly-Thr [Psi (Me, Me) pro]-OH (I):

Fmoc-Gly-Cl (10.6g, 33.6 mmol, 1.0 eq.) was reacted with N, O-Bis(trimethylsilyl)-L-threonine trimethylsilyl ester (11.27g, 1.0 eq.) as described in Example-4, except that triethyl amine was used as the base in place of diisopropylethylamine and obtained Fmoc-Gly-(O-trimethylsilyl)-L-threonine trimethylsilyl ester (23 g) which also contained triethylamine hydrochloride. It was reacted with 2-methoxypropene as described in Example-5 resulting in
Fmoc-Gly-Thr [Psi (Me, Me) pro]-OH (I) in 68.2% yield with 98.7% purity by HPLC.

## Claims

1. A process for the preparation of Fmoc-Gly-Thr [Psi (Me, Me) pro]-OH
having the formula (I), 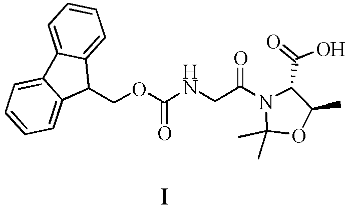 comprising:
(a) reacting Fmoc-glycyl chloride having the formula (II), 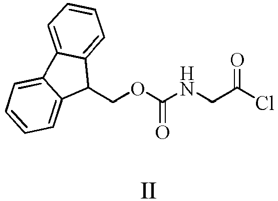 with N, O-Bis(trimethylsilyl)-L-threonine trimethylsilyl ester having the formula (III), in the presence of a base in a non-aqueous organic solvent, 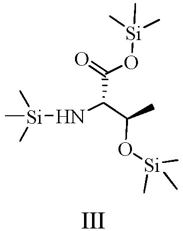
(b) treating the solution containing the reaction mixture from step(a) with water followed by aqueous hydrochloric acid resulting in, at the biphasic surface, precipitation of Fmoc-Gly-Thr-OH (IV), and 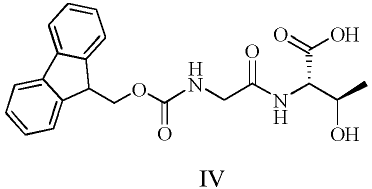
(c) reacting Fmoc-Gly-Thr-OH (IV) with a cyclizing agent, using 2-methyltetrahydro-furan as solvent, at a temperature of 45°C to 75°C in the presence of an acid catalyst to provide Fmoc-Gly-Thr [Psi (Me, Me) pro]-OH of the formula (I).

2. The process as claimed in claim 1(a) wherein the base is triethyl amine or diisopropylethylamine.

3. The process as claimed in claim 1(a) wherein the non-aqueous solvent is methylene chloride.

4. The process as claimed in claim 1(c) wherein the cyclizing agent is 2-methoxy propene or 2,2-dimethoxypropane.

5. The process as claimed in claim 1(c) wherein the acid catalyst is pyridinium p-toluene sulfonate.

6. A process for the preparation of Fmoc-Gly-Thr [Psi (Me, Me) pro]-OH
having the formula (I), 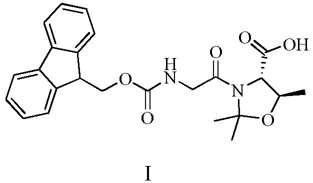 comprising:
(a) reacting Fmoc-glycyl chloride having the formula (II), with N, O-Bis(trimethylsilyl)-L-threonine trimethylsilyl ester having the formula (III), in the presence of a base in a non-aqueous organic solvent to obtain Fmoc- Gly-(O-trimethylsilyl)-Threonine - trimethylsilyl ester of the formula (V), 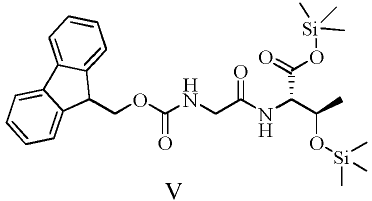
(b) reacting Fmoc-Gly-(O-trimethylsilyl)-Threonine-trimethylsilyl ester of the formula (V) with a cyclizing agent, using 2-methyltetrahydrofuran as solvent, at a temperature of 55°C to 75°C in the presence of an acid catalyst to provide Fmoc-Gly-Thr [Psi (Me, Me) pro]-OH of the formula (I).

7. The process as claimed in claim 6(a) wherein the base is triethyl amine or diisopropylethylamine.

8. The process as claimed in claim 6(a) wherein the organic solvent is methylene chloride.

9. The process as claimed in claim 6(b) wherein the cyclizing agent is 2-methoxy propene or 2,2-dimethoxypropane.

10. The process as claimed in claim 6(c) wherein the acid catalyst is pyridinium p-toluene sulfonate.
